# EUROPEAN PATENT SPECIFICATION

(11) **EP 4 701 536 B1**
(45) Date of publication and mention of the grant of the patent: **28.10.2026**
(21) Application number: 24722495.9
(22) Date of filing: 24.04.2024
(51) Int. Cl.: A61B 8/00, A61B 8/08

(54) **GUIDED CARDIAC ULTRASOUND IMAGING TO MINIMIZE APICAL FORESHORTENING**
GEFÜHRTE KARDIALE ULTRASCHALLBILDGEBUNG ZUR MINIMIERUNG VON APIKALER VERKÜRZUNG
IMAGERIE ULTRASONORE CARDIAQUE GUIDÉE POUR RÉDUIRE À UN MINIMUM LE RACCOURCISSEMENT APICAL

(30) Priority: 27.04.2023 US 202363462362 P
(43) Date of publication of application: 04.03.2026
(73) Proprietor: Koninklijke Philips N.V., 5656 AE Eindhoven (NL)
(72) Inventor: MUKADDIM, Rashid Al, 5656 AG Eindhoven (NL); ERKAMP, Ramon Quido, 5656 AG Eindhoven (NL); BHARAT, Shyam, 5656 AG Eindhoven (NL)
(74) Representative: Philips Intellectual Property & Standards
(86) International application number: PCT/EP2024/061144
(87) International publication number: WO 2024/223599

(56) References cited:
- US-A1- 2022 211 347
- US-A1- 2022 273 260
- SMISTAD ERIK ET AL: "Real-Time Automatic Ejection Fraction and Foreshortening Detection Using Deep Learning", IEEE TRANSACTIONS ON ULTRASONICS, FERROELECTRICS, AND FREQUENCY CONTROL, IEEE, USA, vol. 67, no. 12, 16 March 2020 (2020-03-16), pages 2595 - 2604, XP011822059, ISSN: 0885-3010, [retrieved on 20201123], DOI: 10.1109/TUFFC.2020.2981037

## Description

### TECHNICAL FIELD

The present disclosure relates generally to ultrasound imaging. In particular, an ultrasound system provides guidance to acquire ultrasound images with that minimize foreshortening, such as apical foreshortening in ultrasound images of a left ventricle of the heart.

### BACKGROUND

Physicians use many different medical diagnostic systems and tools to monitor a subject's health and diagnose and treat medical conditions. Ultrasound imaging systems are widely used for medical imaging and measurement. The ultrasound transducer probe may include an array of ultrasound transducer elements that transmit acoustic waves into a subject's body and record acoustic waves reflected and backscattered from the internal anatomical structures within the subject's body, which may include tissues, blood vessels, and internal organs. The transmission and reception of acoustic waves, along with various beamforming and processing techniques, create an image of the subject's internal anatomical structures.

Ultrasound imaging is a safe, useful, and in some applications, non-invasive tool for diagnostic examination, interventions, and/or treatment. Ultrasound imaging can be used to diagnose a wide variety of medical conditions. When imaging various aspects of a patient's heart, accurate probe placement is essential to optimizing the views of the ultrasound images acquired. For example, ultrasound images may be used to quantify metrics such as ejection fraction (EF), stroke volume (SV), and global longitudinal strains (GLS). Incorrect probe placement during an imaging procedure can lead to improper views and, by extension, inaccurate measurements of the particular region of interest of the patient and may lead to improper diagnoses. A lack of expertise, time, or resources can lead to poor quality ultrasound images and inaccurate measurements.

US 2022/273260 A1 describes an ultrasound imaging method includes acquiring low-resolution volume data with an ultrasound probe during the process of acquiring high-resolution slice data of a plane with the ultrasound probe.

### SUMMARY

Aspects of the present disclosure are systems, devices, and methods for guiding ultrasound imaging to minimize foreshortening. Foreshortening can occur when the ultrasound imaging plane does not extend through the actual/true/anatomical apex of the anatomy that is being imaged. For example, apical foreshortening can occur when the ultrasound imaging plane does not extend through the actual/true/anatomical apex of the left ventricle in the heart. Aspects of the present disclosure advantageously assist the user of an ultrasound imaging system to correctly position an ultrasound imaging probe to acquire images with that minimize foreshortening, such as apical foreshortening in ultrasound images of the left ventricle. This advantageously allows inexperienced users and/or users in point-of-care settings, non-ideal imaging settings, etc., to obtain better ultrasound images. Ultrasound images with less foreshortening can be further processed to generate more accurate measurement of relevant features of the patient anatomy, such as ejection fraction (EF), stroke volume (SV), and global longitudinal strains (GLS) associated with the heart.

In some aspects, a user of the ultrasound imaging system is directed to acquire ultrasound images. The ultrasound imaging system analyzes the received images with a deep learning network and determines if the images were obtained at the correct intercostal space and with the correct probe angle. If the images were not obtained at the correct intercostal space, the system outputs guidance to the user directing them to move the probe to the correct intercostal space and additional images are received. If the images were acquired with the correct intercostal space but with an incorrect probe angle, the system outputs guidance to the user to maintain the position of the probe, but to vary the angle of the probe. As the user moves the probe as indicated by the system, the quality of the received images improves until the user obtains an ultrasound image at the correct intercostal space and with the correct angle such that the apical foreshortening of the image is minimal.

In some aspects, a user of the ultrasound imaging system is directed to acquire ultrasound images at multiple intercostal spaces. The ultrasound imaging system then analyzes these images and assigns each image an apical foreshortening indicator metric (AFIM). The AFIM value for each image is then compared and the image with the smallest AFIM is selected as a reference image with the AFIM value selected as a reference AFIM value. The ultrasound imaging system then outputs guidance to the user to position the probe at the intercostal space corresponding to the reference AFIM to acquire additional images. As each image is received, the ultrasound imaging system calculates an AFIM value for the image and compares the new value to the reference AFIM value. If the new AFIM value is less than the reference AFIM value, the new AFIM value is set as the reference AFIM value. If, however, the new AFIM value is not less than the reference AFIM value, the reference AFIM value is not updated. The ultrasound imaging system repeats this process until the user terminates image acquisition or until the reference AFIM is decreased below a threshold value.

In exemplary aspect, an ultrasound system is provided. The ultrasound system includes a processor configured for communication with a display, a transducer array of a handheld ultrasound probe, and a memory, wherein the processor is configured to: control the transducer array to obtain a plurality of ultrasound images corresponding to one or more views of a patient anatomy; determine a foreshortening metric corresponding to one or more ultrasound images of the plurality of ultrasound images; output a foreshortening indicator representative of the foreshortening metric to the display; compare the foreshortening metric to one or more predetermined criteria; and in response to the foreshortening metric not satisfying the one or more predetermined criteria, output, to the display, guidance to a user of the ultrasound system to adjust an orientation of the handheld ultrasound probe.

In some aspects, the foreshortening indicator comprises: a plurality of discrete regions respectively associated with different values of the foreshortening metric; and a graphical element aligned with one of the plurality of regions, based on a value of the foreshortening metric. In some aspects, the foreshortening indicator comprises: a continuous spectrum of different values of the foreshortening metric; and a graphical element at a location along the continuous spectrum that is based on a value of the foreshortening metric. In some aspects, in response to the foreshortening metric satisfying the one or more predetermined criteria, the processor is configured to store the one or more ultrasound images in the memory. In some aspects, in response to the foreshortening metric not satisfying the one or more predetermined criteria, the processor is configured to: control the transducer array to obtain an additional plurality of ultrasound images; calculate an updated foreshortening metric corresponding to the additional plurality of images; and modify the foreshortening indicator based on the updated foreshortening metric. In some aspects, the processor is configured to: compare the updated foreshortening metric to the one or more predetermined criteria; and modify the guidance to the user based on the comparison between the updated foreshortening metric and the one or more predetermined criteria. In some aspects, the foreshortening metric comprises a first component relating to probe position and a second component relating to probe tilt. In some aspects, to compare the foreshortening metric to the one or more predetermined criteria, the processor is further configured to: compare the first component relating to the probe position to a desired probe position; and compare the second component relating to the probe tilt to a desired probe tilt. In some aspects, in response to the first component relating to the probe position not matching the desired probe position, the guidance to the user comprises a message to adjust a position of the handheld ultrasound probe. In some aspects, the message comprises movement of the handheld ultrasound probe to a different intercostal space in a superior direction or an inferior direction. In some aspects, in response to the first component relating to the probe position matching the desired probe position and the second component relating to the probe tilt not matching the desired probe tilt, the guidance to the user comprises a message to adjust an angle of the handheld ultrasound probe while maintaining a position of the handheld ultrasound probe. In some aspects, satisfying the one or more predetermined criteria comprises the first component matching the desired probe position and the second component matching the desired probe tilt. In some aspects, the one or more predetermined criteria comprises a threshold value of the foreshortening metric. In some aspects, to control the transducer array to obtain a plurality of ultrasound images corresponding to the one or more views of the patient anatomy, the processor is further configured to: control the transducer array to obtain a first plurality of ultrasound images while the handheld ultrasound probe is at a first position corresponding to a first view of the patient anatomy; control the transducer array to obtain a second plurality of ultrasound images, while the handheld ultrasound probe is at a second position corresponding to a second view of the patient anatomy; and control the transducer array to obtain a third plurality of ultrasound images, while the handheld ultrasound probe is at a third position corresponding to a third view of the patient anatomy. In some aspects, the patient anatomy comprises a heart, the first view corresponds to a first intercostal space, the second view corresponds to a second intercostal space superior to the first intercostal space, and the third view corresponds to a third intercostal space inferior to the first intercostal space. In some aspects, to calculate the foreshortening metric corresponding to the one or more of the plurality of ultrasound images, the processor is further configured to: calculate a first foreshortening metric associated with the first plurality of ultrasound images; calculate a second foreshortening metric associated with the second plurality of ultrasound images; and calculate a third foreshortening metric associated with the third plurality of ultrasound images. In some aspects, the processor is further configured to: compare the first foreshortening metric, the second foreshortening metric, and the third foreshortening metric; and assign one of the first foreshortening metric, the second foreshortening metric, or the third foreshortening metric to be a historical foreshortening metric. In some aspects, one or more predetermined criteria comprise the historical foreshortening metric.

In an exemplary aspect, an ultrasound system for guiding a user to obtain optimized ultrasound images is provided. The ultrasound system may include a handheld ultrasound probe comprising a transducer array; a display; a memory; and a processor configured for communication with the transducer array, the display, and the memory, wherein the processor is configured to: control the transducer array to obtain a first plurality of ultrasound images corresponding to one or more views of a patient anatomy; calculate a foreshortening metric corresponding to one or more ultrasound images of the plurality of ultrasound images; output a foreshortening indicator representative of the foreshortening metric to the display; compare the foreshortening metric to one or more predetermined criteria; and in response to the foreshortening metric not satisfying the one or more predetermined criteria, iteratively output guidance to the user to adjust the orientation of the handheld ultrasound probe, obtain an additional plurality of ultrasound images, calculate an additional foreshortening metric corresponding to the additional plurality of ultrasound images, update the foreshortening indicator based on the additional foreshortening metric, and compare the additional foreshortening metric to the one or more predetermined criteria until the updated foreshortening metric satisfies the one or more predetermined criteria.

Additional aspects, features, and advantages of the present disclosure will become apparent from the following detailed description.

### BRIEF DESCRIPTION OF THE DRAWINGS

Illustrative aspects of the present disclosure will be described with reference to the accompanying drawings, of which:
Fig. 1 is a schematic diagram of an ultrasound imaging system, according to aspects of the present disclosure.
Fig. 2 is a schematic diagram of a processor circuit, according to aspects of the present disclosure.
Fig. 3 is a flow diagram of a method of obtaining ultrasound images and guiding a user of the ultrasound imaging system to position an ultrasound imaging probe, according to aspects of the present disclosure.
Fig. 4A is a diagrammatic view of a graphical user interface displaying user guidance for obtaining ultrasound images, according to aspects of the present disclosure.
Fig. 4B is a diagrammatic view of a graphical user interface displaying user guidance for obtaining ultrasound images, according to aspects of the present disclosure.
Fig. 4C is a diagrammatic view of a graphical user interface displaying user guidance for obtaining ultrasound images, according to aspects of the present disclosure.
Fig. 5 is a flow diagram of a method of obtaining ultrasound images and guiding a user of the ultrasound imaging system to positions an ultrasound imaging probe, according to aspects of the present disclosure.
Fig. 6A is a diagrammatic view of a graphical user interface displaying user guidance for obtaining ultrasound images, according to aspects of the present disclosure.
Fig. 6B is a diagrammatic view of a graphical user interface displaying user guidance for obtaining ultrasound images, according to aspects of the present disclosure.
Fig. 6C is a diagrammatic view of a graphical user interface displaying user guidance for obtaining ultrasound images, according to aspects of the present disclosure.
Fig. 7 is a schematic diagram of a deep learning algorithm, according to aspects of the present disclosure.
Fig. 8 is a is a schematic diagram of a convolutional neural network (CNN) configuration, according to aspects of the present disclosure.
Fig. 9 is a diagrammatic view of a graphical user interface displaying user guidance for probe position, according to aspects of the present disclosure.
Fig. 10 is a diagrammatic view of a graphical user interface displaying user guidance for probe angle, according to aspects of the present disclosure.

### DETAILED DESCRIPTION

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

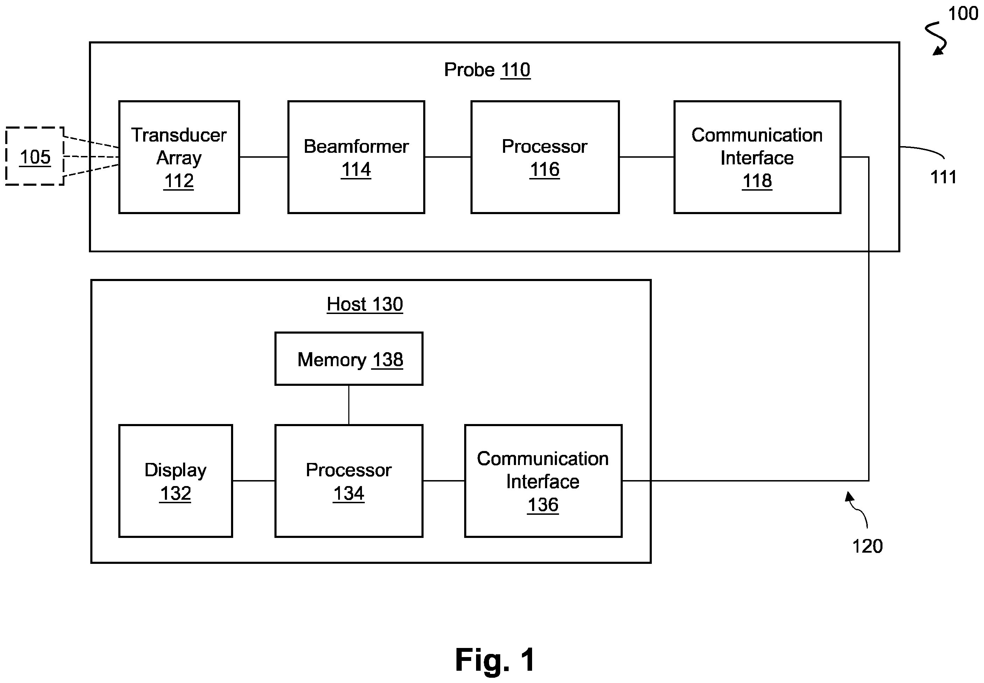

Fig. 1 is a schematic diagram of an ultrasound imaging system 100, according to aspects of the present disclosure. The system 100 is used for scanning an area or volume of a subject's body. A subject may include a patient of an ultrasound imaging procedure, or any other person, or any suitable living or non-living organism or structure. The system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The system 100 may be used to assist a sonographer in performing an ultrasound scan. The scan may be performed in a at a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound system 100 may then analyze the ultrasound image to identify various parameters associated with the acquisition of the image such as the scan window, the probe orientation, the subject position, and/or other parameters. The system 100 may then store the image and these associated parameters in the memory 138. At a subsequent imaging procedure, the system 100 may retrieve the previously acquired ultrasound image and associated parameters for display to a user which may be used to guide the user of the system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

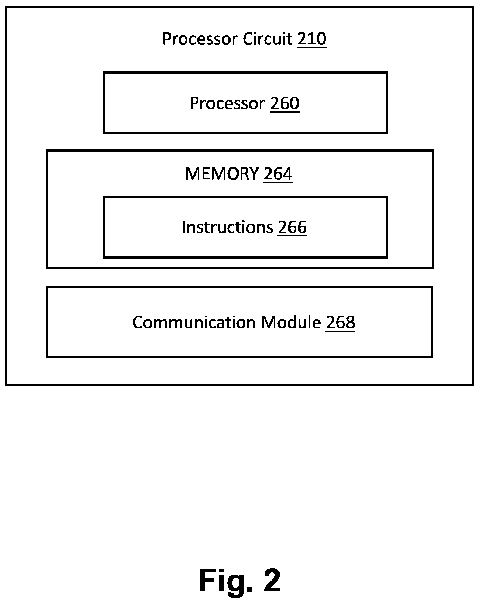

Fig. 2 is a schematic diagram of a processor circuit, according to aspects of the present disclosure. One or more processor circuits can be configured to carry out the operations described herein. The processor circuit 210 may be implemented in the probe 110, the host system 130 of Fig. 1, or any other suitable location. For example, the processor 116 of the probe 110 can be part of the processor circuit 210. For example, the processor 134 and/or the memory 138 can be part of the processor circuit 210. In an example, the processor circuit 210 may be in communication with the transducer array 112, beamformer 114, communication interface 122, communication interface 136, and/or the display 132, as well as any other suitable component or circuit within ultrasound system 100. As shown, the processor circuit 210 may include a processor 260, a memory 264, and a communication module 268. These elements may be in direct or indirect communication with each other, for example via one or more buses.

The processor 260 may include a CPU, a GPU, a DSP, an application-specific integrated circuit (ASIC), a controller, an FPGA, another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration. The processor 260 may also include an analysis module as will be discussed in more detail hereafter. The analysis module may implement various machine learning algorithms and may be a hardware or a software implementation. The processor 260 may additionally include a preprocessor in either hardware or software implementation. The processor 260 may execute various instructions, including instructions stored on a non-transitory computer readable medium, such as the memory 264.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In some instances, the memory 264 includes a non-transitory computer-readable medium. The memory 264 may store instructions 266. The instructions 266 may include instructions that, when executed by the processor 260, cause the processor 260 to perform the operations described herein with reference to the probe 110 and/or the host 130 (Fig. 1). Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 266 may include various aspects of a preprocessor, machine learning algorithm, convolutional neural network (CNN) or various other instructions or code. In some aspect, the memory 264 may be or include a non-transitory computer readable medium.

The communication module 268 can include any electronic circuitry and/or logic circuitry to facilitate direct or indirect communication of data between the processor circuit 210, the probe 110, and/or the host 130. In that regard, the communication module 268 can be an input/output (I/O) device. In some instances, the communication module 268 facilitates direct or indirect communication between various elements of the processor circuit 210 and/or the probe 110 (Fig. 1) and/or the host 130 (Fig. 1).

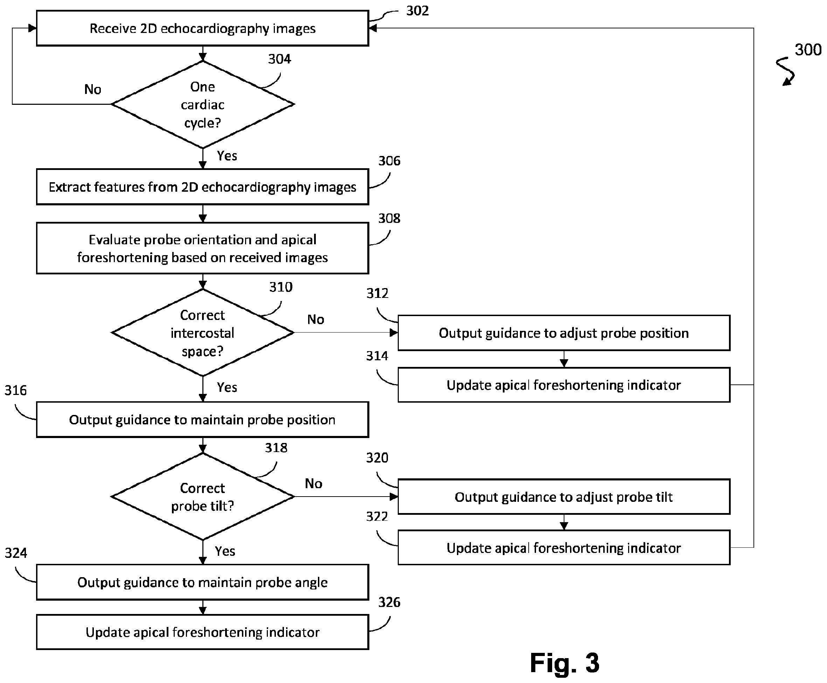

Fig. 3 is a flow diagram of a method of obtaining ultrasound images and guiding a user of the ultrasound imaging system to positions an ultrasound imaging probe, according to aspects of the present disclosure. As illustrated, the method 300 includes a number of enumerated steps, but aspects of the method 300 may include additional steps before, after, or in between the enumerated steps. In some aspects, one or more of the enumerated steps may be omitted, performed in a different order, or performed concurrently. The steps of the method 300 can be carried out by any suitable component within the system 100 and all steps need not be carried out by the same component. In some aspects, one or more steps of the method 300 can be performed by, or at the direction of, a processor circuit, including, e.g., the processor 116 (Fig. 1), the processor 134 (Fig. 1), the processor 260 (Fig. 2) or any other suitable component.

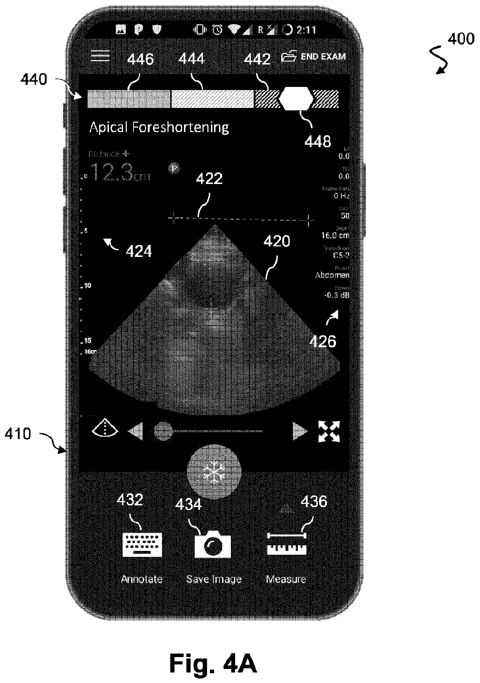

Aspects of the method 300 may describe methods of providing guidance to non-expert ultrasound imaging system users to acquire high quality ultrasound images. As will be explained hereafter, these ultrasound images may include views of a region of a patient's anatomy. Various aspects of the method 300 and Fig. 3 will be described with reference to Figs. 4A-4C which are diagrammatic views of a graphical user interface 400 displaying user guidance for obtaining ultrasound images, according to aspects of the present disclosure.

In some aspects, the method 300 may be implemented by the ultrasound imaging system 100, e.g., by the processor 134 and/or the processor 210, to ensure that the user acquires ultrasound images with minimal foreshortening. To do so, the ultrasound imaging system 100 may ensure that the orientation of the probe is correct during image acquisition. The probe orientation may be described as including at least two components: (1) the intercostal position of the probe or the intercostal space through the ultrasound probe acquires images, and (2) the angle of the ultrasound transducer probe. In some aspects, the probe orientation may include other components such as the tilt of the ultrasound transducer or the longitudinal position of the probe along one intercostal space.

At step 302, the method 300 includes receiving 2D ultrasound/echocardiography images. Receiving the ultrasound images can include a processor controlling a transducer array to obtain the ultrasound images. In some aspects, the ultrasound images depict anatomy of the heart, such as a chamber of the heart (left ventricle, left atrium, right ventricle, right atrium). The ultrasound images can be a series of image frames over time (e.g., ultrasound video or video clip). The ultrasound images can be live ultrasound images, received during live imaging (e.g., in real time or near real time). The images received at the step 302 may include any suitable type of ultrasound images. For example, in some aspects, the images received may include B-mode images, Doppler images, and/or combinations thereof. Additionally, any suitable type of ultrasound imaging system may be used to acquire the ultrasound images at step 302. For example an ultrasound imaging system may be similar to the ultrasound imaging system 100 described with reference to Fig. 1.

At step 304, the method 300 includes verifying that the images received at step 302 correspond to at least one full cardiac cycle. At step 304, the ultrasound imaging system 100 may ensure that the images received at the step 302 are sufficient to perform the subsequent steps of the method 300. Verifying that the images received at step 302 correspond to at least one full cardiac cycle may include comparing the ultrasound images to corresponding reference images, as well as determining whether particular features or characteristics are present within ultrasound images. In some aspects, verifying that the images received at step 302 may include comparing a number of ultrasound images received to an expected number or a threshold number. The expected or threshold number of images may be calculated based on various factors such as the heart rate of the patient, frame rate of the ultrasound imaging system, or other factors. In some aspects, a user of the ultrasound imaging system may verify that the images received at step 302 correspond to a full cardiac cycle or the ultrasound system may automatically perform this verification, for example via a machine learning network of the ultrasound imaging system 100. For example, a machine learning network may be trained to analyze and/or compare received ultrasound images and determine whether the images correspond to a full cardiac cycle.

At step 306, the method 300 includes extracting features from the 2D echocardiography images received at step 302. Step 306 will be described together with step 308. It is noted, that the step 306 may be optionally performed. For instance, at step 308, the method includes evaluating the probe orientation and apical foreshortening of the images received at step 302 based on the received images. This evaluation may be performed based on the images themselves and/or based on features extracted from the images. For example, when images are received at step 302, the ultrasound probe is at a particular position. At step 308, the ultrasound system is tasked with determining what the probe position is and whether the probe position corresponds to an ideal probe position in terms of the extent of apical foreshortening observed in the acquired images. In some aspects, the ultrasound imaging system may automatically determine the position of the ultrasound probe based on the images alone, without extracting particular features of the images. For example, the ultrasound imaging system may include a machine learning network that is trained to determine the probe position based on received ultrasound images. For instance, the machine learning network may receive, as an input, the images received at step 302. These images may correspond to different views of the patient anatomy. Some images may depict, for example, a left ventricle of the heart of a patient from various angles. The machine learning network may be trained to distinguish between images of the left ventricle from different angles.

In aspects in which the ultrasound imaging system performs step 306, and features are extracted from the images received at step 302, the machine learning network of the ultrasound system may receive as an input extracted features of the received images. The ultrasound imaging system 100 may be configured to extract any suitable features from the ultrasound images received. In particular, the ultrasound imaging system 100 may, by any suitable machine learning network or algorithm or by various image processing techniques, identify any suitable features within the images. By way of example, if the particular region of interest of the patient anatomy includes the patient's heart, extracted features may include the visibility of mitral valves and apex, left ventricle length, sphericity of the ventricle, ratio of left ventricle to left atrium area or any other suitable features. The presence, shape, or location of any of these features within the images received at step 302 may then be used to determine the probe orientation associated with the received images.

In some aspects, the ultrasound system 100 may be configured to receive as inputs to determine an orientation of the ultrasound transducer probe, both the ultrasound images themselves as well as extracted features of the ultrasound images. In some aspects, determining the probe orientation may include determining an intercostal space of the probe. In other words, determining the probe orientation may include determining by which space between the ribs of the patient the probe was oriented during image acquisition. In some aspects, ensuring that the received ultrasound images include minimal foreshortening may include both determining whether the ultrasound probe is position at the appropriate intercostal space and determining whether the angle of the ultrasound transducer probe is correct. In some aspects, as shown in Fig. 3, the ultrasound transducer system 100 may first determine whether the ultrasound imaging probe is positioned at the appropriate intercostal space and provide the user with guidance to move the probe to the appropriate intercostal space if necessary, and then determine if the probe angle is correct and provide corresponding guidance if necessary, as explained below.

At step 310, the method 300 includes determining whether the ultrasound transducer probe is positioned at the appropriate intercostal space. In some aspects, step 310 may include evaluating an input or output of the machine learning network described with reference to step 308. For example, an output of the machine learning network of step 308 may include a designation of an intercostal space. This intercostal space may be associated with a particular number, side of the patient body, or any other suitable nomenclature. In some aspects, if the ultrasound imaging system 100 determines that the intercostal space is not correct, the system 100 proceeds to the step 312 of the method 300, as shown in Fig. 3. However, if the intercostal space is correct, the ultrasound imaging system 100 may proceed to the step 316 of the method 300.

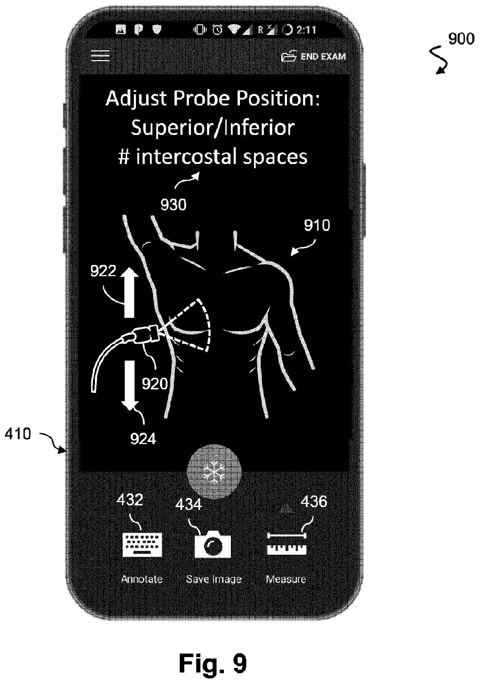

At step 312, the method 300 includes outputting guidance to a user of the ultrasound imaging system 100 to adjust the position of the ultrasound imaging probe. In some aspects, this guidance may include guidance that the position needs to be moved in any direction. In some aspects, the guidance to the user may include a recommended direction of the ultrasound imaging probe. For example, the ultrasound imaging system 100 may recommend that the user move the probe in an inferior or superior direction. In some aspects, the ultrasound imaging system 100 may provide guidance to a user to move the ultrasound imaging probe to the other side of the patient. In some aspects, the ultrasound imaging system 100 may output guidance to the user to move the ultrasound imaging probe in a superior or inferior direction as well as provide a recommendation of the number of intercostal spaces to move the probe. For example, the probe may determine that the current probe position must be moved in a superior direction by two intercostal spaces, and may provide guidance to that effect. Any of the guidance output to the user at step 312 may include any guidance shown and described with reference to Fig. 9.

At step 314, the method 300 includes updating an apical foreshortening indicator. With reference to Fig. 4A, an apical foreshortening indicator 440 may be shown on an example graphical user interface 400.

As previously explained, Fig. 4A is a diagrammatic view of a graphical user interface 400 displaying user guidance for obtaining ultrasound images, according to aspects of the present disclosure. As shown, the graphical user interface 400 may be presented on the display of a device 410. The device 410 may be any suitable device. In the example shown in Fig. 4A, the device 410 may be a smart phone. However, in other implementations, the device 410 may alternatively be a tablet, a computer, including a mobile laptop or desktop computer, a host system of an ultrasound imaging system, or any other suitable device. In some aspects, the method 300 and corresponding graphical user interface 400 may be particularly suited to a mobile application used by a novice user in a non-ideal procedural setting, in which the guidance from the ultrasound imaging system 100 may assist the user in obtaining ultrasound images of ideal quality and minimal apical foreshortening.

As shown, the graphical user interface 400 includes an ultrasound image 420, a measurement indicator 422, and a scale 424. In some aspects, the ultrasound image 420 may correspond to a live image received in a point-of-care setting in an ultrasound imaging procedure. In some aspects, the ultrasound image 420 may correspond to an image previously acquired, including an image from a previous procedure, or an image received by the ultrasound system 100 at an earlier stage of the current procedure. The measurement indicator 422 and scale 424 may assist a user in determining distances within the ultrasound image 420 and may assist a user in making various diagnoses as needed.

The graphical user interface 400 also includes an annotation button 432, a save image button 434, and a measure button 436. In some aspects, the measurement indicator 422 and scale 424 may be presented to the user within the graphical user interface 400 in response to the user selecting the measure button 436. In response to the user selecting the save image button 434, the ultrasound image 420 may be stored in a memory of the device 410 or in another memory associated with the device 410. For example, the device 410 may be in wired or wireless communication with a storage device, such as an external memory or cloud storage server which may store the ultrasound image 420 and any associated data as well as any suitable data associated with the imaging procedure or patient. In some aspects, the graphical user interface 400 may be modified to include various displays, buttons, or indicators allowing the user to provide notes associated with, or overlaid on, the ultrasound image 420. In some aspects, the graphical user interface may also be modified to allow the user to highlight, outline, or otherwise annotate the ultrasound image 420 and/or its corresponding features, metrics or indicators.

Additionally shown in Fig. 4A is a number of metrics 426. These metrics may include or correspond to any suitable data. For example, the metrics 426 may include metrics or data associated with the ultrasound image 420. For example, the metrics 426 may include measurements of the image 420, including distance measurements, volume measurements, or area measurements. In some aspects, the metrics 426 may include data, measurements, or settings of the ultrasound system 100 more broadly during the particular imaging procedure. For example, the metrics 426 may include a frame rate, a time duration of the procedure, a gain setting of the ultrasound imaging system 100, a depth setting of the ultrasound imaging system 100, the type of transducers of the probe, a pattern, number, or group of transducers used for the imaging procedure, a description of the region of interest of the patient or a view of the ultrasound images, descriptions of the features extracted from the ultrasound image 420, a power setting of the ultrasound imaging system 100 or any other suitable metrics, settings, or characteristics.

Fig. 4A additionally includes the apical foreshortening indicator 440. In some aspects, the apical foreshortening indicator 440 may inform a user of the system 100 as to the degree of apical foreshortening observed in the received ultrasound images, such as the image 420, as well as which components of the probe orientation are correct or incorrect. For example, the apical foreshortening indicator 440 may include three discrete regions: a region 442, a region 444, and a region 446. The apical foreshortening indicator 440 additionally includes a graphical element 448 overlaid over the any of the regions 442, 444, and/or 446. The position of the graphical element 448 may provide different guidance to the user. For example, referring again to Fig. 3, if the ultrasound imaging system 100 determines at step 310 that the probe position is not at the correct intercostal position, at step 314, the apical foreshortening indicator 440 (Fig. 4A) may be updated such that the graphical element 448 is positioned within the region 442. In that regard, the region 442 may correspond to an incorrect probe position, or the probe being positioned at an incorrect intercostal space.

In some aspects, the position of the graphical element 448 may provide additional guidance for a user. For example, if the probe position is far from the correct intercostal space (e.g., 4 or 5 intercostal spaces away), the graphical element 448 may be positioned in the right section of the region 442. Whereas, if the probe position is incorrect, but close to the correct intercostal space (e.g., 1 intercostal space away), the graphical element 448 may be positioned in the left section of the region 442. In that regard, if the user of the ultrasound imaging system 100 initially positions the probe at the incorrect intercostal space, the ultrasound imaging system 100 may update the graphical user interface with the graphical element 448 within the region 442. As the user moves the probe to different intercostal spaces, the graphical element 448 may be moved in real time such that if the user observes the graphical element 448 moving to the right within the region 442, the user knows that he or she is moving the probe in the wrong direction and can reverse directions. As he or she does so, the graphical element 448 may be updates to move to the left.

This updating in real time of the apical foreshortening indicator 440, including the position of the graphical element 448 is also shown and described in Fig. 3. In particular, after the apical foreshortening indicator 440 is updated to move the graphical element 448 to the appropriate position at step 314, the ultrasound imaging system 100 reverts back to step 302 of the method 300 at which additional images are received. Steps 304-310 are then performed again and the ultrasound imaging system 100 may again update the apical foreshortening indicator 440 depending on the new position of the ultrasound imaging probe.

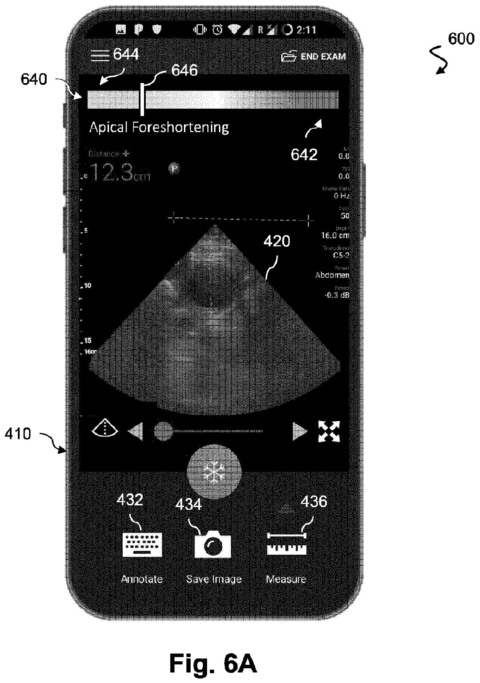

It is noted, that in some aspects, the apical foreshortening indicator 440 may be of any suitable appearance or type. For example, an additional embodiment is shown and described with reference to the apical foreshortening indicator 640 of Figs. 6A-6C hereafter. However, additional types of apical foreshortening indicators are expected. For example, graphical element 448 may be of any suitable appearance or the appearance of the graphical element 448 may be modified to reflect the position of the probe in any suitable way. In some aspects, the apical foreshortening indicator 440 may alternatively not include the graphical element 448. For example, if the ultrasound imaging system 100 determines at step 310 that the probe is not in the correct intercostal position, the region 442 may be highlighted or otherwise emphasized over the regions 444 and 446 such that the user is made aware that the probe is not in the correct intercostal position.

Again referencing Fig. 3, if the ultrasound system 100 determines that the probe is positioned at the correct intercostal space at step 310, the method 300 progress to step 316. At step 316, the method 300 includes outputting guidance to the user to maintain the probe position. This guidance may be of any suitable type, including a visual element including text, shapes, symbols, colors, or any other visual appearance, as well as auditory or haptic feedback of any suitable type. In some aspects, the guidance may include any guidance shown and described with reference to Fig. 9. In some aspects, the guidance to maintain the probe position may be provided in the form of updating the apical foreshortening indicator at steps 322 or 324 explained hereafter.

At step 318, the method 300 includes determining whether the ultrasound transducer probe is held at the appropriate angle. In some aspects, step 318, like step 310 previously described, may include evaluating an input or output of the machine learning network described with reference to step 308. For example, an output of the machine learning network of step 308 may include a designation or quantification of the angle of the ultrasound imaging probe. This quantification may include an angle in degrees or radians or may include multiple coordinates corresponding to the angle, tilt, fan, or rock of the ultrasound transducer probe in any direction. In some aspects, determining whether the angle of the probe is correct may include comparing the determining angle of the probe to a previously stored, ideal angle. For example, the ultrasound imaging transducer may determine that the angle of the probe is within an acceptable range of the ideal angle, including for example, within a certain number of degrees or radians over or under the ideal angle. In some aspects, if the ultrasound imaging system 100 determines that the angle is not correct, for example, by falling outside of this range, the system 100 proceeds to the step 320 of the method 300, as shown in Fig. 3. However, if the probe angle is correct, the ultrasound imaging system 100 may proceed to the step 324 of the method 300.

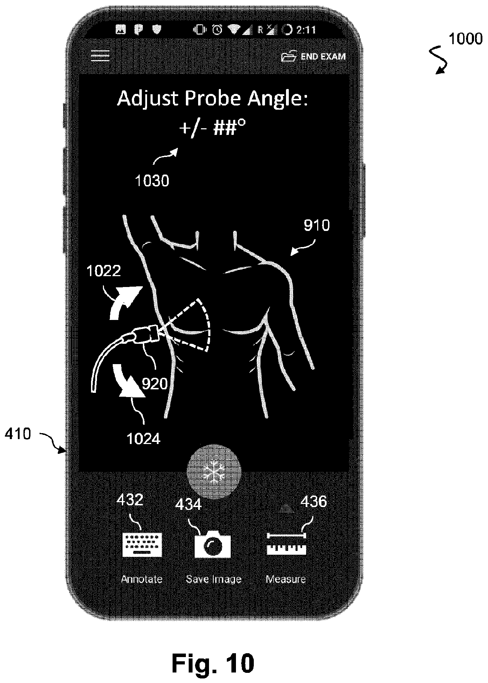

At step 320, the method 300 includes outputting guidance to a user of the ultrasound imaging system 100 to adjust the angle of the ultrasound imaging probe. In some aspects, this guidance may include guidance that the angle needs to be adjusted in any direction. In some aspects, the guidance to the user may include a recommended direction of angle adjustment for the ultrasound imaging probe. In some aspects, the ultrasound imaging system 100 may output guidance to the user to adjust the angle of the probe by a specified amount, such as by a specified number of degrees or radians. This guidance may be output in any suitable way. For example, this guidance may be output as text overlaid over any features of the graphical user interface 400 shown in Figs. 4A-4C. The guidance provided at step 320 may include any of the guidance shown and described with reference to Fig. 10.

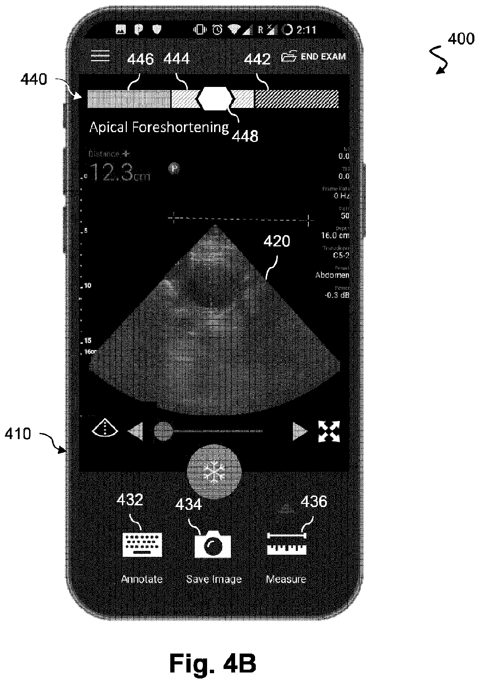

At step 322, the method 300 includes updating an apical foreshortening indicator. With reference to Fig. 4B, the apical foreshortening indicator 440 as updated at step 322 may be shown. If the probe position is determined to be correct (e.g., at step 310), but the probe angle is determined to be incorrected (e.g., at step 318), the apical foreshortening indicator 440 may be updated as shown in Fig. 4B such that the graphical element 448 is moved to the region 444. In that regard, the region 444 may correspond to an indication to the user that the probe is in the correct intercostal position (and the probe position should be maintained) but that the angle is not correct and should be adjusted.

Similar to the region 442 of Fig. 4A, in some aspects, the position of the graphical element 448 within the region 444 may provide additional information regarding the probe orientation, and particularly the probe angle. For example, if the current probe angle is severely incorrect, the position of the graphical element 448 may be moved to the right section of the region 444. However, if the position of the graphical element 448 is close to the ideal probe angle, but not within the threshold range, the graphical element 448 may be moved to the left section of the region 444. Thus, a user may quickly determine, in real time, whether the user is adjusting the probe angle in the correct direction or not and adjust accordingly. Similarly, if the apical foreshortening indicator 440 does not include the graphical element 448, the region 444 may alternatively be highlighted or otherwise emphasized, as described with reference to the region 442.

Referring again to Fig. 3, after the apical foreshortening indicator is updated at step 322, for example, by moving the graphical element 448 into the region 444, the method 300 reverts again to step 302 and additional images are acquired. After the subsequent steps are performed, if the ultrasound imaging system 100 determines that the probe position has been maintained at the correct intercostal space at step 310 and the probe angle is correct at 318, the ultrasound imaging system 100 may proceed to step 324.

At step 324, the method 300 includes outputting guidance to the user to maintain the probe angle. This guidance may be of any suitable type, including a visual element including text, shapes, symbols, colors, or any other visual appearance, as well as auditory or haptic feedback of any suitable type. In some aspects, the guidance provided may include any of the guidance shown and described with reference to Fig. 10. In some aspects, the guidance to maintain the probe angle may be provided in the form of updating the apical foreshortening indicator at step 326.

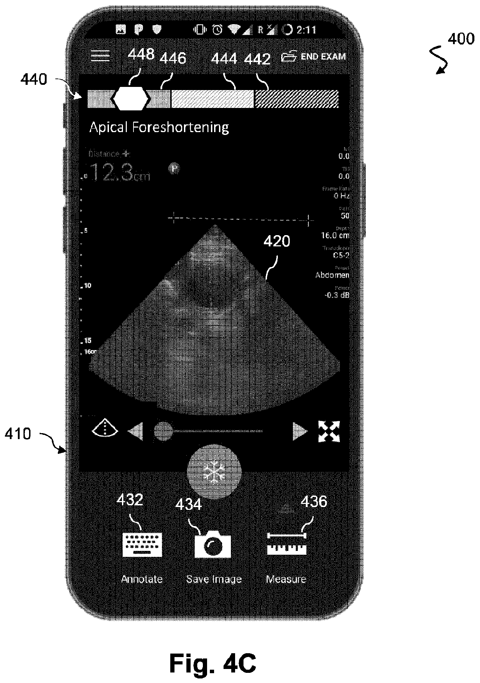

At step 326, the method 300 includes updating the apical foreshortening indicator 440. With reference to Fig. 4C, if the probe position and probe angle are both correct, the apical foreshortening indicator 440 may be updated such that the graphical element 448 is moved within the region 446. In that regard, the region 446 may correspond to the probe orientation, including the position and angle, being correct. As described with reference to the regions 444 and 442 previously, in aspects, in which the apical foreshortening indicator 440 does not include the graphical element 448, the region 446 may be highlighted or emphasized in any suitable way to convey that the probe position and angle are both correct.

In some aspects, the regions 442, 444, and 446 may be visually differentiated from one another in any suitable way. For example, the regions 442, 44, and 446 may be displayed with different colors. In some aspects, the region 442 may correspond to a red color, the region 44 may correspond to a yellow color, and the region 446 may correspond to a green color. Any other suitable colors may also be used. In some aspects, the regions 442, 444, and 446 may alternatively be visually differentiated from one another in any other way, for example, by using different patterns, outlines, or any other visual characteristics.

In some aspects, the method 300 can include performing additional processing of the ultrasound images that have been obtained with minimized apical foreshortening (e.g., according to steps 302-326). The additional processing of the ultrasound images can include automatically identifying anatomy within the images, quantifying anatomical values within the images (ejection fraction (EF), stroke volume (SV), and global longitudinal strains (GLS), etc. In some aspects, the processor automatically performs these steps after the ultrasound images with minimized apical foreshortening is stored in memory, without receiving a user input to initiate identification of the anatomy and/or quantifying the anatomical values. The method can also include outputting graphical/visual representations associated with the identified anatomy and/or the quantified anatomical values. Using ultrasound images with minimized apical foreshortening advantageously improves the accuracy of identifying the anatomy and/or quantifying the anatomical values.

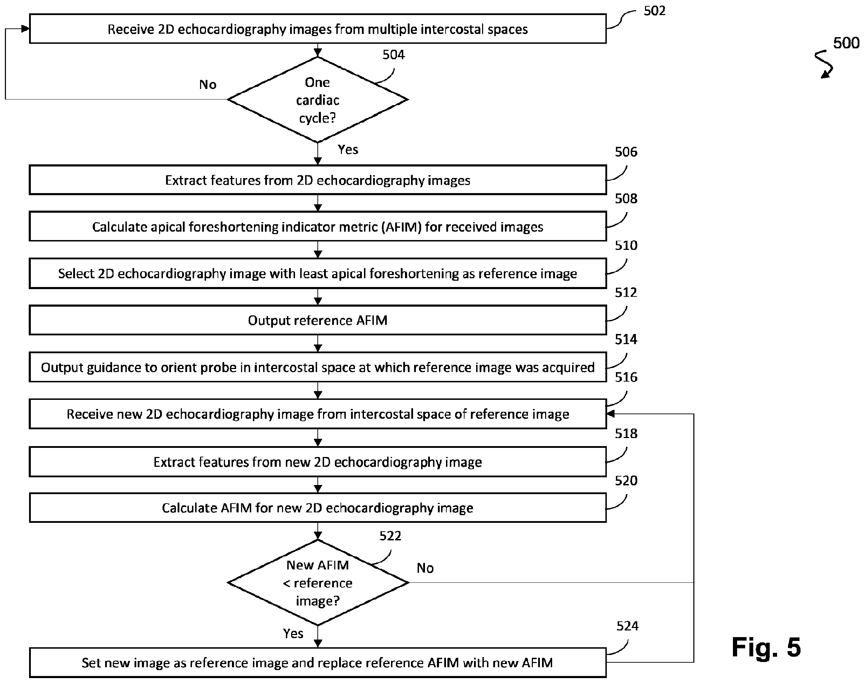

Fig. 5 is a flow diagram of a method 500 of obtaining ultrasound images and guiding a user of the ultrasound imaging system to position an ultrasound imaging probe, according to aspects of the present disclosure. In some aspects, the method 500 may be similar to the method 300. As illustrated, the method 500 includes a number of enumerated steps, but aspects of the method 500 may include additional steps before, after, or in between the enumerated steps. In some aspects, one or more of the enumerated steps may be omitted, performed in a different order, or performed concurrently. The steps of the method 500 can be carried out by any suitable component within the system 100 and all steps need not be carried out by the same component. In some aspects, one or more steps of the method 500 can be performed by, or at the direction of, a processor circuit, including, e.g., the processor 116 (Fig. 1), the processor 134 (Fig. 1), the processor 260 (Fig. 2) or any other suitable component.

Aspects of the method 500 will be described with reference to Figs. 6A-6C. Figs. 6A-6C are diagrammatic views of a graphical user interface 600 displaying user guidance for obtaining ultrasound images, according to aspects of the present disclosure.

At step 502, the method 500 includes receiving 2D ultrasound/echocardiography images from multiple intercostal spaces. Receiving the ultrasound images can include a processor controlling a transducer array to obtain the ultrasound images. In some aspects, the ultrasound images depict anatomy of the heart, such as a chamber of the heart (left ventricle, left atrium, right ventricle, right atrium). The ultrasound images can be a series of image frames over time (e.g., ultrasound video or video clip). The ultrasound images can be live ultrasound images, received during live imaging (e.g., in real time or near real time). The images received at the step 302 may include any suitable type of ultrasound images. For example, in some aspects, the images received may include B-mode images, Doppler images, and/or combinations thereof. Additionally, any suitable type of ultrasound imaging system may be used to acquire the ultrasound images at step 502. For example, an ultrasound imaging system may be similar to the ultrasound imaging system 100 described with reference to Fig. 1.

In some aspects, the ultrasound imaging system 100 will direct the user to obtain images from multiple intercostal spaces. For example, the ultrasound imaging system 100 may output, to a screen display in communication with the processor, guidance to the user to obtain images from multiple intercostal spaces. This guidance may be of any suitable type, including, for example, text, graphical elements, or any other suitable type of guidance.

In some aspects, the ultrasound imaging system 100 may perform any of the steps of the method 300 described previously to identify the intercostal space of all images received at step 502. For example, the ultrasound imaging system 100, after determining that a received image or a plurality of received images was obtained at a particular intercostal space, may output guidance to a user to move the ultrasound probe in a superior or inferior direction, such as an moving the probe by one intercostal space superiorly or inferiorly, and to obtain additional images. In some aspects, the ultrasound imaging system 100 may direct the user to obtain images from a predetermined number of intercostal spaces, such as two, three, four, or more. These intercostal spaces may be directly adjacent to one another or spaced according to any suitable pattern or plan.

At step 504, the method 500 includes verifying that the images received at step 502 correspond to at least one full cardiac cycle. In some aspects, step 504 may include verifying that enough images were received at step 502 such that a full cardiac cycle's worth of images was received at each intercostal space. At step 504, the ultrasound imaging system 100 may ensure that the images received at the step 502 are sufficient to perform the subsequent steps of the method 500. Verifying that the images received at step 502 correspond to at least one full cardiac cycle may include comparing the ultrasound images to corresponding reference images, as well as determining whether particular features or characteristics are present within ultrasound images. In some aspects, verifying that the images received at step 502 may include comparing a number of ultrasound images received to an expected number or a threshold number. The expected or threshold number of images may be calculated based on various factors such as the heart rate of the patient, frame rate of the ultrasound imaging system, or other factors. In some aspects, a user of the ultrasound imaging system may verify that the images received at step 502 correspond to a full cardiac cycle or the ultrasound system may automatically perform this verification, for example via a machine learning network of the ultrasound imaging system 100. For example, a machine learning network may be trained to analyze and/or compare received ultrasound images and determine whether the images correspond to a full cardiac cycle.

At step 506, the method 500 includes extracting features from the 2D echocardiography images received at step 502. Step 506 will be described together with step 508. It is noted that the step 506 may be optionally performed. For instance, at step 508, the method includes calculating an apical foreshortening indicator metrics (AFIM) for the images received at step 502. This calculation may be performed based on the images themselves and/or based on features extracted from the images. For example, calculating an AFIM may include any suitable techniques or methods described with reference to method 300. In that regard, calculating the AFIM may be based at least partially on a position of the ultrasound probe as determined by the ultrasound imaging system 100 and/or an angle of the ultrasound probe as determined by the ultrasound imaging system 100.

In some aspects, the AFIM may be any suitable value. For example, the AFIM may be a value within a range, such as a numerical value within a range of 1-5 as a non-limiting example. In some aspects, the AFIM may be a percentage, a ratio, rank, such as a letter or number rank, or any other suitable type of metric.

In some aspects, at step 508, the ultrasound imaging system 100 may be configured to calculate an AFIM for each intercostal space. For example, at step 502, images received at the same intercostal space may be designated as such by annotation and/or by grouping these images together and storing them in a memory. The ultrasound imaging system 100 may calculate an AFIM for each set of images within a group corresponding to each intercostal space. In some aspects, the ultrasound imaging system 100 may calculate an AFIM for each received image. In some aspects, the ultrasound imaging system 100 may calculate an AFIM for each received image and calculate a mean AFIM for all images received corresponding to one intercostal space. In that regard, an AFIM may be assigned to an intercostal space.

In some aspects, the value of the AFIM may correspond to the amount of apical foreshortening observed in the corresponding image or set of images. In that regard, a high AFIM value may correspond to a large amount of apical foreshortening observed in the corresponding image or set of images and may not be ideal, while a low AFIM value may correspond to a small amount of apical foreshortening observed in the corresponding image or set of images.

At step 510, the method 500 includes selecting a 2D echocardiography image with the least apical foreshortening as a reference image. In that regard, the calculated AFIM for all images received at step 502 may be compared, regardless of the intercostal space at which each image was acquired. The ultrasound system 100 may select the image with the smallest AFIM as the image with the least apical foreshortening. The ultrasound imaging system 100 may then annotate or otherwise designate the selected image as a reference image. In some aspects, the reference image may alternatively be referred to as a historical image.

In some aspects, the ultrasound imaging system 100 may alternatively compare the AFIM for each intercostal space. In that regard, AFIM for each set of images corresponding to each intercostal space may be compared. The ultrasound imaging system 100 may then select the lowest AFIM as a reference AFIM and designate the corresponding intercostal space as the reference intercostal space or target intercostal space. The reference AFIM may alternatively be referred to as a historical AFIM.

At step 512, the method 500 includes outputting the reference AFIM. The ultrasound imaging system 100 may additionally output to the display the reference image, reference AFIM, or reference intercostal space selected. This output may be of any suitable type.

Fig. 6A provides an example of the output of the reference AFIM. Turning to Fig. 6A, an example graphical user interface 600 is shown. The graphical user interface 600 includes an apical foreshortening indicator 640. The apical foreshortening indicator 640 may be similar to the apical foreshortening indicator 440 previously described. In that regard, the apical foreshortening indicator 640 includes a graphical element 646. The graphical element 646 may indicate the reference AFIM. For example, the apical foreshortening indicator 640 may correspond to a spectrum of AFIM values. In some aspects, the right region 642 of the apical foreshortening indicator 640 may correspond to a high AFIM value. Conversely, the left region 644 may correspond to a low AFIM value and positions between these two regions may correspond to a continuous spectrum of AFIM values between the two extremes. In some aspects, the apical foreshortening indicator 640 may be accompanied by a scale such that a user may determine an absolute AFIM value corresponding to the graphical element 646 by observing the position of the graphical element 646 along the scale.

At step 514, the method 500 includes outputting guidance to the user to orient the ultrasound imaging probe in the intercostal space at which the reference image was acquired. In that regard, the ultrasound imaging system 100 may output visual guidance by, for example, pictures, stylized graphics, or any other suitable visual elements pointing to the intercostal space of the patient. Such an output may also include textual guidance. The guidance provided at step 514 may include any of the guidance shown and described with reference to Fig. 9.

At step 516, the method 500 includes receiving new 2D echocardiography images from the intercostal space of the reference image. After the user has positioned the probe at the selected intercostal space according to the guidance of step 514, the ultrasound imaging system 100 may begin to receive one or more additional images.

At step 518, the method 500 includes extracting features from the newly received 2D echocardiography image received at step 516. In some aspects, extracting features from the new image at step 518 may include any of the same procedures or methods as described at step 506 of the method 500 and/or step 306 of the method 300.

At step 520, the method 500 includes calculating an AFIM for the newly received 2D echocardiography image. Aspects of calculating an AFIM at step 520 may include any of the procedures or methods described at step 508 of the method 500.

At step 522, the method 500 includes determining if the new AFIM value is less than the AFIM value of the reference image. Step 522 may include comparing the AFIM value calculated at step 520 to the reference AFIM value calculated at step 508. If the new AFIM value calculated at step 520 is greater than the reference AFIM value calculated at step 508, the apical foreshortening indicator 640 may be updated and the method 500 reverts back to step 516.

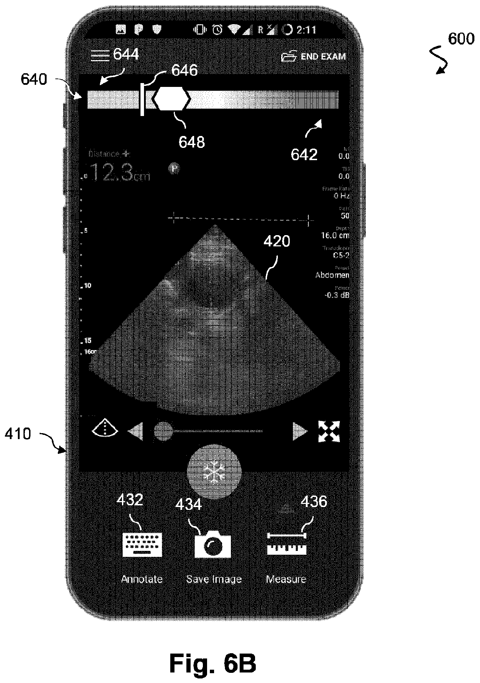

Fig. 6B provides an example graphical user interface which may be displayed to a user after the ultrasound imaging system 100 determines that the new AFIM value is greater than the reference AFIM value. Turning to Fig. 6B, because the new AFIM value was not less than the reference AFIM value, the graphical element 646 corresponding to the reference AFIM value is not changed. The graphical element 646 remains in the same position along the apical foreshortening indicator 640.

In some aspects, the apical foreshortening indicator may be updated to include a graphical element 648. The graphical element 648 may be positioned along the apical foreshortening indicator 640 to indicate the AFIM value of the image received at step 516. As shown in Fig. 6B, because the AFIM value of the new image is less than the reference AFIM value, the graphical element 648 is positioned to the right of the graphical element 646. After the graphical user interface 600 is updated, the steps 516 through 522 may be performed again.

If, at step 522, the ultrasound imaging system 100 determines that the AFIM value of a newly received image (e.g., received at step 516) is less than the reference AFIM value, the apical foreshortening indicator 640 again be updated and the method 500 proceeds to step 524.

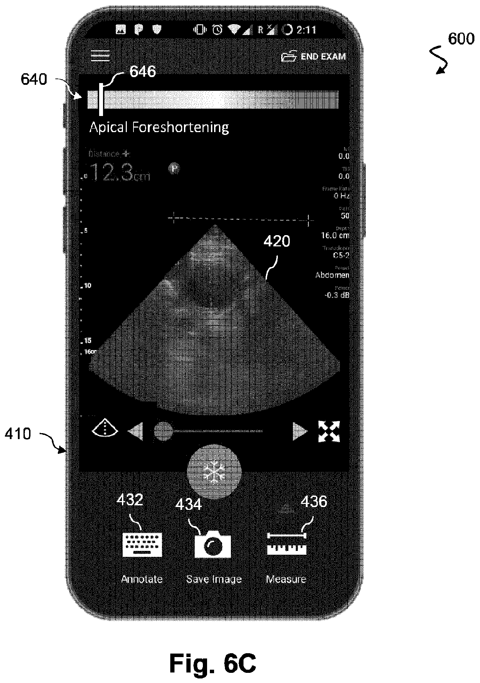

Fig. 6C provides an example of the graphical user interface 600 after the ultrasound imaging system 100 determines that an AFIM value of a newly received image is less than the reference AFIM value at step 522. Turning to Fig. 6C, the graphical element 646 may be moved to the left as compared to the location of the graphical element 646 in Figs. 6A or 6B. In that regard, the ultrasound imaging system 100 may designate the newly received image as a new reference image and the AFIM value of the newly received image as a new reference AFIM value.

As shown in Fig. 5, after the step 524 is performed, the method 500 may again revert to step 516 and additional images may be received. In that regard, the ultrasound imaging system 100 may iteratively perform steps 516-524 of the method 500, thus improving the reference AFIM value. This may proceed until the user ends the procedure. In some aspects, the procedure may end when the reference AFIM value is less than a predetermined threshold value. In some aspects, the ultrasound imaging system 100 may be configured to designate the reference image as the image to be used to perform any suitable calculations or measurements for the ultrasound imaging procedure. In that regard, the reference image may be the image with least apical foreshortening resulting in measurements that are as accurate as possible, despite any inexperience of the user or non-ideal procedure setting.

In some aspects, the method 500 can include performing additional processing of the ultrasound images that have been obtained with minimized apical foreshortening (e.g., according to steps 502-524). The additional processing of the ultrasound images can include automatically identifying anatomy within the images, quantifying anatomical values within the images (ejection fraction (EF), stroke volume (SV), and global longitudinal strains (GLS), etc. In some aspects, the processor automatically performs these steps after the ultrasound images with minimized apical foreshortening is stored in memory, without receiving a user input to initiate identification of the anatomy and/or quantifying the anatomical values. The method can also include outputting graphical/visual representations associated with the identified anatomy and/or the quantified anatomical values. Using ultrasound images with minimized apical foreshortening advantageously improves the accuracy of identifying the anatomy and/or quantifying the anatomical values.

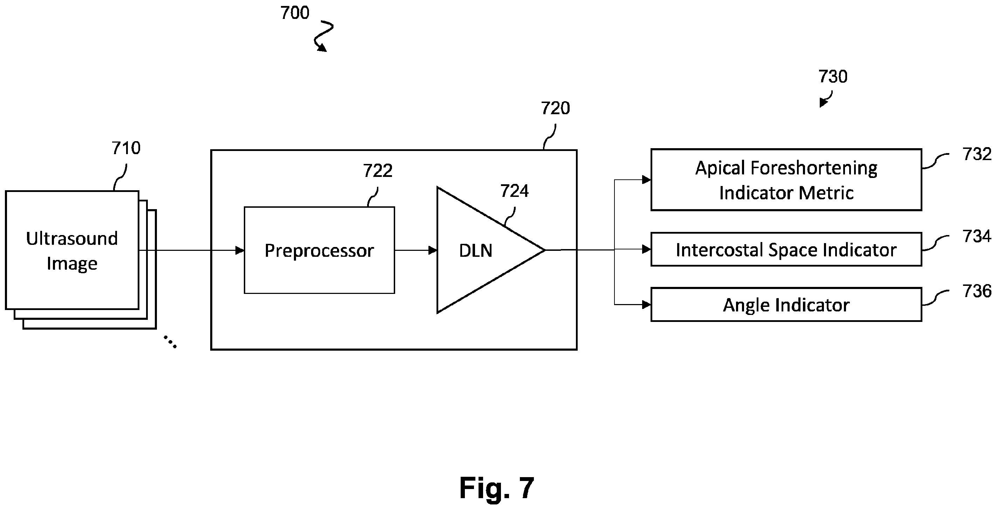

Fig. 7 is a schematic diagram of a machine learning algorithm 700, according to aspects of the present disclosure. The machine learning algorithm 700 can also be referred to as an artificial intelligence framework. In that regard, the artificial intelligence framework 700 may include a machine learning network and various aspects of the framework may be performed by the processor 134 and/or the processor circuit 210 and may include instructions similar to the instructions 266 previously described. The machine learning algorithm 700 may also be a deep learning algorithm in some embodiments. The processor circuit 210 may be configured to use a machine learning network to determine the intercostal space at which an ultrasound image is received, determine a probe angle at which an ultrasound image was received, and/or calculate an apical foreshortening metric. The embodiment of the artificial intelligence framework 700 shown in Fig. 7 includes various input ultrasound images 710. The ultrasound images 710 may be received from various sources such as the ultrasound imaging system 100, a picture archiving and communication system (PACS), or other image storage system. The ultrasound images 710 may be received or arranged in a chronological order. For example, the ultrasound images 710 may have been obtained by the imaging system 100 during an ultrasound imaging procedure. The framework 700 may include an analysis module 720 with a preprocessor 722 and a deep learning network 724. In some embodiments, the analysis module 720 can be implemented in the host 130 (Fig. 1) and/or the processor circuit 210 (Fig. 2). The artificial intelligence framework 700 may generate multiple outputs 730. The machine learning algorithm 700 may be trained to calculate apical foreshortening metrics as output 732, an intercostal space indicator as output 734, and an angle indicator as output 736.

Training the machine learning algorithm 700 may be accomplished with various different techniques. In one embodiment, training the deep learning network may be accomplished by creating a large dataset of sample ultrasound images depicting varying apical foreshortening including varying intercostal spaces and probe angles. The sample images may additionally be obtained from a large number of patients. The deep learning network may be trained using multiple annotated ultrasound images. Each of the multiple annotated ultrasound images include an annotation corresponding to any of the outputs 732, 734, and/or 736.

The analysis module 720 can include a preprocessor 722, which can include hardware (e.g., electrical circuit components) and/or software algorithms (e.g., executed by a processor). The preprocessor 722 may perform various functions to adjust, filter, or otherwise modify received input images 710 before transmitting the input images 710 to the deep learning network 724. For example, the preprocessor 722 may manipulate incoming images. The preprocessor 722 may perform various image processing steps for both training and prediction purposes. The preprocessor 722 may perform various tasks such as changing the contrast of the image, size of the image, resolution, orientation, geometry, cropping, spatial transformation, resizing, normalizing, histogram modification or various other procedures to assist deep learning network 724 to work more efficiently or accurately.

The deep learning network 724 may receive as an input the processed images 710 from the preprocessor 722. The deep learning network 724 can include hardware (e.g., electrical circuit components) and/or software algorithms (e.g., executed by a processor). The deep learning network 724 may then identify various parameters associated with the received ultrasound images 710.

The deep learning network 724 can include a convolutional neural network (CNN) in some embodiments. For example, the CNN can be or include a multi-class classification network, or an encoder-decoder type network. In some instances, the analysis module can implement any kind of classification process, such as a random forest algorithm, a classification tree approach, a convolutional neural network or any type of deep learning network. In some instances, the analysis module can implement a statistical model or random forest algorithm based on image or ultrasound backscatter derived parameters instead of a deep learning network.

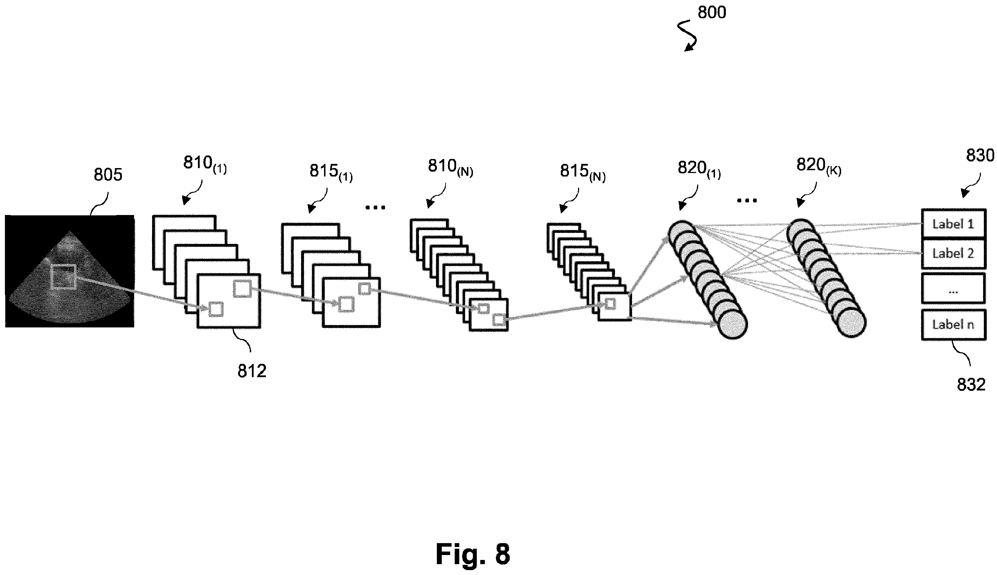

Fig. 8 is a schematic diagram of a convolutional neural network (CNN) configuration 800, according to aspects of the present disclosure. For example, the CNN configuration 800 can be implemented as the deep learning network 724 (Fig. 7). In an embodiment, the configuration 800 may perform a classification task. For example, a convolutional neural network (CNN) may provide classification labels for each pixel of an ultrasound image or signal. The configuration 800 may be of any suitable type and may include any suitable type or number of layers including but not limited to convolutional layers, fully connected layers, flatten vectors, or any other techniques or implementations of artificial intelligence systems. The embodiments shown and/or described with reference to Fig. 8 can be scaled to include any suitable number of CNNs (e.g., about 2, 3 or more). The configuration 800 can be trained for identification of any of the outputs described above.

The CNN may include a set of N convolutional layers 810 where N is any positive integer, each layer followed by a pooling layer 815. The CNN may also include a set of K fully connected layers 820, where K may be any positive integer. In one embodiment, the fully connected layers 820 include at least two fully connected layers 820. The convolutional layers 810 are shown as 810(1) to 810(N). The pooling layers 815 are shown as 815(1) to 815(N). The fully connected layers 820 are shown as 820(1) to 820(K). Each convolutional layer 810 may include a set of filters 812 configured to extract features from an input 805 (e.g., ultrasound images or other additional data). The convolutional layers 810 may include convolutional kernels of different sizes and strides. The values N and K and the size of the filters 812 may vary depending on the embodiments. In some instances, the convolutional layers 810(1) to 810(N), the pooling layers 815(1) to 815(N), and the fully connected layers 820(1) to 820(K-1) may utilize a sigmoid, rectified non-linear (ReLU), leaky ReLU, softmax, or hyperbolic tangent activation function. The pooling layers 815 may include max pooling or average pooling techniques. The fully connected layers 820 may gradually shrink the high-dimensional output to a dimension of the prediction result (e.g., the classification output 830). Thus, the fully connected layers 820 may also be referred to as a classifier. In some embodiments, the fully convolutional layers 810 may additionally be referred to as perception or perceptive layers. The fully connected layers 820 may downsample and map received information to a finite number of classes 832. In an embodiment, the final fully connected layer 820(K) may be followed by a final classification layer such as softmax to transform the net activations in the final output layer to a series of values that can be interpreted as probabilities.

The classification output 830 may indicate a confidence score or probability for each of a plurality of classes 832, based on the input image 805. In that regard, the CNN 800 can be a multi-class classification network. In an exemplary embodiment, the plurality of classes 832 may include the apical foreshortening metric, the intercostal space, and the probe angle.

In an embodiment in which the deep learning network includes an encoder-decoder network, the network may include two paths. One path may be a contracting path, in which a large image, such as the image 805, may be convolved by several convolutional layers 810 such that the size of the image 805 changes in depth of the network. The image 805 may then be represented in a low dimensional space, or a flattened space. From this flattened space, an additional path may expand the flattened space to the original size of the image 805. In some embodiments, the encoder-decoder network implemented may also be referred to as a principal component analysis (PCA) method. In some embodiments, the encoder-decoder network may segment the image 805 into patches.

In some embodiments, multiple convolutional neural networks may be implemented to identify different characteristics of received ultrasound images. For example, one CNN may be trained to identify an apical foreshortening metric, a separate CNN may be trained to identify the intercostal space, and an additional CNN may identify the probe angle. Any CNN may be trained to identify any one of these characteristics within a received ultrasound image, including just one, some, or all of these characteristics.

In some embodiments, aspects of the machine learning network may include a post-processing step. The post-processing step may combine outcomes of one or more classification or learning algorithms and/or apply a logic based on the size and spacing of pixels of an ultrasound image This post-processing step may be an additional algorithm to all other algorithms. This post-processing step may base a scoring of an ultrasound image or individual pixels of an ultrasound image both on quantitative analysis of the label clusters and published studies, literature, or empirical results from experts in the field. In some embodiments, this post-processing step may be an artificial algorithm itself.

Fig. 9 is a diagrammatic view of a graphical user interface 900 displaying user guidance for probe position, according to aspects of the present disclosure. As shown, the graphical user interface 900 may be displayed on the screen of the device 410. The graphical user interface 900 may include a graphical representation of a patient 910 as well as a graphical representation of an ultrasound imaging probe 920. In some aspects, the position of the imaging probe 920 may be selected by the imaging system relative to the representation of the patient 910 such that the position of the probe 920 indicates to the user the correct position of the probe relative to the patient. As also shown in Fig. 9, the graphical user interface 900 may include directive arrows 922 and 924. In some aspects, if the ultrasound imaging system determines that the probe 920 should be moved in a superior direction, the arrow 922 may be shown on the display. If, however, the ultrasound imaging system determines that the probe 920 should be moved in an inferior direction, the error 924 may be displayed. Similarly, various text guidance 930 may be included within the display. The text guidance 930 may indicate to the user the type of movement for the ultrasound imaging probe 920. In the example shown in Fig. 9, the type of movement may be adjusting probe position. In some examples, the text 930 may include a direction to move the ultrasound imaging probe. Specifically, the text 930 may include the term superior if the probe is to be moved in the superior direction or may include the term inferior if the probe is to be moved in an inferior direction. In addition, the text 930 may include a number of intercostal spaces by which the user should move the ultrasound imaging probe. For example, if the ultrasound imaging system determines that the probe 920 is incorrectly placed and should be moved in a superior direction by two intercostal spaces, the text 930 may include the term "superior" and the number two, or the terms "two intercostal spaces." Similar directives may be included within the text 930. In addition, while arrows corresponding to the inferior and superior directions are shown in Fig. 9, it is expected that additional directions may be denoted by similar arrows. For example, an arrow may be positioned next to the probe 920 indicating movement of the probe from side to side or to the opposite side of the patient.

Fig. 10 is a diagrammatic view of a graphical user interface displaying user guidance for probe angle, according to aspects of the present disclosure. As shown, the graphical user interface 1000 may be displayed on the screen of the device 410. The graphical user interface 1000 may similarly include the graphical representation of the patient 910 as well as a graphical representation of the ultrasound imaging probe 920. In some aspects, the angle of the imaging probe 920 may be selected by the imaging system relative to the representation of the patient 910 such that the angle of the probe 920 indicates to the user the correct angle of the probe relative to the patient. As also shown in Fig. 10, the graphical user interface 1000 may include directive arrows 1022 and 1024. In some aspects, if the ultrasound imaging system determines that the angle of the probe 920 should be adjusted in a direction corresponding to the arrow 1022 may be shown on the display. In some aspects, the direction corresponding to the arrow 1022 may be selected as a positive direction. In some aspects, the direction corresponding to the arrow 1024 may be selected as a negative direction. In that regard, if the ultrasound imaging system determines that the angle of the probe 920 should be adjusted in the opposite direction, the arrow 1024 may be displayed. Similarly, various text guidance 1030 may be included within the display. The text guidance 1030 may indicate to the user the type of movement for the ultrasound imaging probe 920. In the example shown in Fig. 10, the type of movement may be adjusting probe angle. In some examples, the text 1030 may include a direction of rotation to move the ultrasound imaging probe. Specifically, the text 1030 may include a positive or negative in degrees or radians. In some aspects, multiple angles may be provided corresponding to multiple axes of rotation. While arrows 1022 and 1024 are shown in Fig. 10, it is expected that additional directions may be denoted by similar arrows.

Persons skilled in the art will recognize that the apparatus, systems, and methods described above can be modified in various ways. Accordingly, persons of ordinary skill in the art will appreciate that the aspects encompassed by the present disclosure are not limited to the particular exemplary aspects described above. In that regard, although illustrative aspects have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound system (100), comprising:
a processor (116) configured for communication with a display (132), a transducer array (112) of a handheld ultrasound probe (110), and a memory (138),
wherein the processor is configured to:
control the transducer array to obtain a plurality of ultrasound images corresponding to one or more views of a patient anatomy;
determine a foreshortening metric corresponding to one or more ultrasound images of the plurality of ultrasound images;
output a foreshortening indicator representative of the foreshortening metric to the display;
compare the foreshortening metric to one or more predetermined criteria; and
in response to the foreshortening metric not satisfying the one or more predetermined criteria, output, to the display, guidance to a user of the ultrasound system to adjust an orientation of the handheld ultrasound probe.

2. The ultrasound system of claim 1, wherein the foreshortening indicator comprises:
a plurality of discrete regions respectively associated with different values of the foreshortening metric; and
a graphical element aligned with one of the plurality of regions, based on a value of the foreshortening metric.

3. The ultrasound system of claim 1, wherein the foreshortening indicator comprises:
a continuous spectrum of different values of the foreshortening metric; and
a graphical element at a location along the continuous spectrum that is based on a value of the foreshortening metric.

4. The ultrasound system of claim 1, wherein, in response to the foreshortening metric satisfying the one or more predetermined criteria, the processor is configured to store the one or more ultrasound images in the memory.

5. The system of claim 1, wherein, in response to the foreshortening metric not satisfying the one or more predetermined criteria, the processor is configured to:
control the transducer array to obtain an additional plurality of ultrasound images;
calculate an updated foreshortening metric corresponding to the additional plurality of images; and
modify the foreshortening indicator based on the updated foreshortening metric.

6. The system of claim 5, wherein the processor is configured to:
compare the updated foreshortening metric to the one or more predetermined criteria; and
modify the guidance to the user based on the comparison between the updated foreshortening metric and the one or more predetermined criteria.

7. The ultrasound system of claim 1, wherein the foreshortening metric comprises a first component relating to probe position and a second component relating to probe tilt.

8. The ultrasound system of claim 7, wherein, to compare the foreshortening metric to the one or more predetermined criteria, the processor is further configured to:
compare the first component relating to the probe position to a desired probe position; and
compare the second component relating to the probe tilt to a desired probe tilt.

9. The ultrasound system of claim 8, wherein, in response to the first component relating to the probe position not matching the desired probe position, the guidance to the user comprises a message to adjust a position of the handheld ultrasound probe.

10. The ultrasound system of claim 9, wherein the message comprises movement of the handheld ultrasound probe to a different intercostal space in a superior direction or an inferior direction.

11. The ultrasound system of claim 8, wherein, in response to the first component relating to the probe position matching the desired probe position and the second component relating to the probe tilt not matching the desired probe tilt, the guidance to the user comprises a message to adjust an angle of the handheld ultrasound probe while maintaining a position of the handheld ultrasound probe.

12. The ultrasound system of claim 8, wherein satisfying the one or more predetermined criteria comprises the first component matching the desired probe position and the second component matching the desired probe tilt.

13. The ultrasound system of claim 1, wherein the one or more predetermined criteria comprises a threshold value of the foreshortening metric.

14. The ultrasound system of claim 1, wherein, to control the transducer array to obtain a plurality of ultrasound images corresponding to the one or more views of the patient anatomy, the processor is further configured to:
control the transducer array to obtain a first plurality of ultrasound images while the handheld ultrasound probe is at a first position corresponding to a first view of the patient anatomy;
control the transducer array to obtain a second plurality of ultrasound images, while the handheld ultrasound probe is at a second position corresponding to a second view of the patient anatomy; and
control the transducer array to obtain a third plurality of ultrasound images, while the handheld ultrasound probe is at a third position corresponding to a third view of the patient anatomy.

15. An ultrasound system (100) for guiding a user to obtain optimized ultrasound images, comprising:
a handheld ultrasound probe (110) comprising a transducer array (112);
a display (132);
a memory (138); and
a processor (116) configured for communication with the transducer array, the display, and the memory, wherein the processor is configured to:
control the transducer array to obtain a first plurality of ultrasound images corresponding to one or more views of a patient anatomy;
calculate a foreshortening metric corresponding to one or more ultrasound images of the plurality of ultrasound images;
output a foreshortening indicator representative of the foreshortening metric to the display;
compare the foreshortening metric to one or more predetermined criteria; and
in response to the foreshortening metric not satisfying the one or more predetermined criteria, iteratively output guidance to the user to adjust the orientation of the handheld ultrasound probe, obtain an additional plurality of ultrasound images, calculate an additional foreshortening metric corresponding to the additional plurality of ultrasound images, update the foreshortening indicator based on the additional foreshortening metric, and compare the additional foreshortening metric to the one or more predetermined criteria until the updated foreshortening metric satisfies the one or more predetermined criteria.

## Patentansprüche

1. Ultraschallsystem (100), umfassend:
einen Prozessor (116), der für Kommunikation mit einem Display (132), einer Wandleranordnung (112) einer handgeführten Ultraschallsonde (110) und einem Speicher (138) konfiguriert ist,
wobei der Prozessor konfiguriert ist:
die Wandleranordnung so zu steuern, dass eine Vielzahl von Ultraschallbildern erhalten wird, die einer oder mehreren Ansichten einer Patientenanatomie entsprechen;
ein Verkürzungsmaß zu bestimmen, das einem oder mehreren Ultraschallbildern der Vielzahl von Ultraschallbildern entspricht;
einen Verkürzungsindikator, der das Verkürzungsmaß darstellt, auf dem Display auszugeben;
das Verkürzungsmaß mit einem vorbestimmten Kriterium oder mehreren vorbestimmten Kriterien zu vergleichen; und
falls das Verkürzungsmaß das eine vorbestimmte Kriterium oder die mehreren vorbestimmten Kriterien nicht erfüllt, auf dem Display eine Anleitung für den Benutzer des Ultraschallsystems auszugeben, um eine Ausrichtung der handgeführten Ultraschallsonde anzupassen.

2. Ultraschallsystem nach Anspruch 1, wobei der Verkürzungsindikator Folgendes umfasst:
eine Vielzahl diskreter Regionen, die jeweils unterschiedlichen Werten des Verkürzungsmaßes zugeordnet sind; und
ein grafisches Element, das auf Basis eines Wertes des Verkürzungsmaßes an einer der Vielzahl von Regionen ausgerichtet ist.

3. Ultraschallsystem nach Anspruch 1, wobei der Verkürzungsindikator Folgendes umfasst:
ein kontinuierliches Spektrum unterschiedlicher Werte des Verkürzungsmaßes; und
ein grafisches Element an einer Stelle entlang des kontinuierlichen Spektrums, die auf einem Wert der Verkürzungsmaß basiert.

4. Ultraschallsystem nach Anspruch 1, wobei der Prozessor so konfiguriert ist, dass er, falls das Verkürzungsmaß das eine vorbestimmte Kriterium oder die mehreren vorbestimmten Kriterien erfüllt, ein oder mehrere Ultraschallbilder im Speicher speichert.

5. System nach Anspruch 1, wobei, falls das Verkürzungsmaß das eine vorbestimmte Kriterium oder die mehreren vorbestimmten Kriterien nicht erfüllt, der Prozessor konfiguriert ist:
die Wandleranordnung zu steuern, um eine zusätzliche Vielzahl von Ultraschallbildern zu erhalten;
ein aktualisiertes Verkürzungsmaß zu berechnen, das der zusätzlichen Vielzahl von Bildern entspricht; und
den Verkürzungsindikator auf Basis des aktualisierten Verkürzungsmaßes zu ändern.

6. System nach Anspruch 5, wobei der Prozessor konfiguriert ist:
das aktualisierte Verkürzungsmaß mit dem einen vorbestimmten Kriterium oder den mehreren vorbestimmten Kriterien zu vergleichen; und
die Anleitung für den Benutzer auf Basis des Vergleichs zwischen dem aktualisierten Verkürzungsmaß und dem einen vorbestimmten Kriterium oder den mehreren vorbestimmten Kriterien zu ändern.

7. Ultraschallsystem nach Anspruch 1, wobei das Verkürzungsmaß eine erste Komponente in Bezug auf eine Sondenposition und eine zweite Komponente in Bezug auf eine Sondenneigung umfasst.

8. Ultraschallsystem nach Anspruch 7, wobei der Prozessor, um das Verkürzungsmaß mit dem einen vorbestimmten Kriterium oder den mehreren vorbestimmten Kriterien zu vergleichen, ferner konfiguriert ist:
die erste Komponente, die sich auf die Sondenposition bezieht, mit einer gewünschten Sondenposition zu vergleichen; und
die zweite Komponente, die sich auf die Sondenneigung bezieht, mit einer gewünschten Sondenneigung zu vergleichen.

9. Ultraschallsystem nach Anspruch 8, wobei die Anleitung für den Benutzer eine Meldung zur Anpassung einer Position der handgeführten Ultraschallsonde umfasst, falls die erste Komponente, die sich auf die Sondenposition bezieht, nicht mit der gewünschten Sondenposition übereinstimmt.

10. Ultraschallsystem nach Anspruch 9, wobei die Meldung Bewegung der handgeführten Ultraschallsonde zu einem anderen Interkostalraum in Aufwärtsrichtung oder Abwärtsrichtung umfasst.

11. Ultraschallsystem nach Anspruch 8, wobei, falls die erste Komponente, die sich auf die Sondenposition bezieht, mit der gewünschten Sondenposition übereinstimmt, und die zweite Komponente, die sich auf die Sondenneigung bezieht, nicht mit der gewünschten Sondenneigung übereinstimmt, die Anleitung für den Benutzer eine Meldung umfasst, einen Winkel der handgeführten Ultraschallsonde anzupassen, während eine Position der handgeführten Ultraschallsonde beibehalten wird.

12. Ultraschallsystem nach Anspruch 8, wobei Erfüllen des einen vorbestimmten Kriteriums oder der mehreren vorbestimmten Kriterien umfasst, dass die erste Komponente mit der gewünschten Sondenposition übereinstimmt und die zweite Komponente mit der gewünschten Sondenneigung übereinstimmt.

13. Ultraschallsystem nach Anspruch 1, wobei das eine vorbestimmte Kriterium oder die mehreren vorbestimmten Kriterien einen Schwellenwert des Verkürzungsmaßes umfassen.

14. Ultraschallsystem nach Anspruch 1, wobei der Prozessor, um die Wandleranordnung zu steuern, um eine Vielzahl von Ultraschallbildern zu erhalten, die der einen oder den mehreren Ansichten der Patientenanatomie entsprechen, ferner konfiguriert ist:
die Wandleranordnung so zu steuern, dass eine erste Vielzahl von Ultraschallbildern erhalten wird, während sich die handgeführte Ultraschallsonde an einer ersten Position befindet, die einer ersten Ansicht der Patientenanatomie entspricht;
die Wandleranordnung so zu steuern, dass eine zweite Vielzahl von Ultraschallbildern erhalten wird, während sich die handgeführte Ultraschallsonde in einer zweiten Position befindet, die einer zweiten Ansicht der Patientenanatomie entspricht; und
die Wandleranordnung so zu steuern, dass eine dritte Vielzahl von Ultraschallbildern erhalten wird, während sich die handgeführte Ultraschallsonde in einer dritten Position befindet, die einer dritten Ansicht der Patientenanatomie entspricht.

15. Ultraschallsystem (100) zur Anleitung eines Benutzers, um optimierte Ultraschallbilder zu erhalten, umfassend:
eine handgeführte Ultraschallsonde (110), die eine Wandleranordnung (112) umfasst;
ein Display (132);
einen Speicher (138); und
einen Prozessor (116), der für Kommunikation mit der Wandleranordnung, dem Display und dem Speicher konfiguriert ist, wobei der Prozessor konfiguriert ist:
die Wandleranordnung so zu steuern, dass eine erste Vielzahl von Ultraschallbildern erhalten wird, die einer oder mehreren Ansichten einer Patientenanatomie entsprechen;
ein Verkürzungsmaß zu berechnen, das einem oder mehreren Ultraschallbildern der Vielzahl von Ultraschallbildern entspricht;
einen Verkürzungsindikator, der das Verkürzungsmaß darstellt, auf dem Display auszugeben;
das Verkürzungsmaß mit einem vorbestimmten Kriterium oder mehreren vorbestimmten Kriterien zu vergleichen; und
falls das Verkürzungsmaß das eine vorbestimmte Kriterium oder die mehreren vorbestimmten Kriterien nicht erfüllt, wiederholt eine Anleitung für den Benutzer auszugeben, um die Ausrichtung der handgeführten Ultraschallsonde anzupassen, eine zusätzliche Vielzahl von Ultraschallbildern zu erhalten, ein zusätzliches Verkürzungsmaß entsprechend der zusätzlichen Vielzahl von Ultraschallbildern zu berechnen, den Verkürzungsindikator auf Basis des zusätzlichen Verkürzungsmaßes zu aktualisieren und das zusätzliche Verkürzungsmaß mit dem einen vorbestimmten Kriterium oder den mehreren vorbestimmten Kriterien zu vergleichen, bis das aktualisierte Verkürzungsmaß das eine vorbestimmte Kriterium oder die mehreren vorbestimmten Kriterien erfüllt.

## Revendications

1. Système ultrasonore (100) comprenant :
un processeur (116) configuré pour communiquer avec un écran (132), un réseau de transducteurs (112) d'une sonde à ultrasons portative (110) et une mémoire (138),
dans lequel le processeur est configuré pour :
commander le réseau de transducteurs afin d'obtenir une pluralité d'images ultrasonores correspondant à une ou plusieurs vues de l'anatomie d'un patient ;
déterminer une mesure de raccourcissement correspondant à une ou plusieurs images ultrasonores parmi la pluralité d'images ultrasonores ;
afficher à l'écran un indicateur de raccourcissement représentatif de la mesure de raccourcissement ;
comparer la mesure de raccourcissement à un ou plusieurs critères prédéterminés ; et
en réponse au fait que la mesure de raccourcissement ne satisfait pas aux un ou plusieurs critères prédéterminés, afficher à l'écran des instructions à l'utilisateur du système ultrasonore pour régler l'orientation de la sonde à ultrasons portative.

2. Système ultrasonore selon la revendication 1, dans lequel l'indicateur de raccourcissement comprend :
une pluralité de régions discrètes associées, respectivement, à différentes valeurs de la mesure de raccourcissement ; et
un élément graphique aligné sur l'une parmi la pluralité de régions, en fonction d'une valeur de la mesure de raccourcissement.

3. Système ultrasonore selon la revendication 1, dans lequel l'indicateur de raccourcissement comprend :
un spectre continu de différentes valeurs de la mesure de raccourcissement ; et
un élément graphique situé à un emplacement le long du spectre continu, basé sur une valeur de la mesure de raccourcissement.

4. Système ultrasonore selon la revendication 1, dans lequel, en réponse au fait que la mesure de raccourcissement satisfait les un ou plusieurs critères prédéterminés, le processeur est configuré pour stocker les une ou plusieurs images ultrasonores dans la mémoire.

5. Système selon la revendication 1, dans lequel, en réponse au fait que la mesure de raccourcissement ne satisfait pas aux un ou plusieurs critères prédéterminés, le processeur est configuré pour :
commander le réseau de transducteurs afin d'obtenir une pluralité supplémentaire d'images ultrasonores ;
calculer une mesure de raccourcissement mise à jour correspondant à la pluralité supplémentaire d'images ; et
modifier l'indicateur de raccourcissement en fonction de la mesure de raccourcissement mise à jour.

6. Système selon la revendication 5, dans lequel le processeur est configuré pour :
comparer la mesure de raccourcissement mise à jour aux un ou plusieurs critères prédéterminés ; et
modifier les instructions destinées à l'utilisateur en fonction de la comparaison effectuée entre la mesure de raccourcissement mise à jour et les un ou plusieurs critères prédéterminés.

7. Système ultrasonore selon la revendication 1, dans lequel la mesure de raccourcissement comprend un premier composant relatif à la position de la sonde et un second composant relatif à l'inclinaison de la sonde.

8. Système ultrasonore selon la revendication 7, dans lequel, pour comparer la mesure de raccourcissement aux un ou plusieurs critères prédéterminés, le processeur est configuré en outre pour :
comparer le premier composant relatif à la position de la sonde à une position de sonde souhaitée ; et
comparer le second composant relatif à l'inclinaison de la sonde à une inclinaison de sonde souhaitée.

9. Système ultrasonore selon la revendication 8, dans lequel, en réponse au fait que le premier composant relatif à la position de la sonde ne correspond pas à la position de sonde souhaitée, les instructions destinées à l'utilisateur comprennent un message pour régler une position de la sonde à ultrasons portative.

10. Système ultrasonore selon la revendication 9, dans lequel le message comprend le déplacement de la sonde à ultrasons portative vers un espace intercostal différent dans une direction supérieure ou une direction inférieure.

11. Système ultrasonore selon la revendication 8, dans lequel, en réponse au fait que le premier composant relatif à la position de la sonde correspond à la position de sonde souhaitée et au fait que le second composant relatif à l'inclinaison de la sonde ne correspond pas à l'inclinaison de sonde souhaitée, les instructions destinées à l'utilisateur comprennent un message pour régler un angle de la sonde à ultrasons portative tout en maintenant une position de la sonde à ultrasons portative.

12. Système ultrasonore selon la revendication 8, dans lequel le fait de satisfaire les un ou plusieurs critères prédéterminés comprend le premier composant correspondant à la position de sonde souhaitée et le second composant correspondant à l'inclinaison de sonde souhaitée.

13. Système ultrasonore selon la revendication 1, dans lequel les un ou plusieurs critères prédéterminés comprennent une valeur seuil de la mesure de raccourcissement.

14. Système ultrasonore selon la revendication 1, dans lequel, pour commander le réseau de transducteurs afin d'obtenir une pluralité d'images ultrasonores correspondant aux une ou plusieurs vues de l'anatomie du patient, le processeur est configuré en outre pour :
commander le réseau de transducteurs afin d'obtenir une première pluralité d'images ultrasonores, tandis que la sonde à ultrasons portative se trouve dans une première position correspondant à une première vue de l'anatomie du patient ;
commander le réseau de transducteurs afin d'obtenir une deuxième pluralité d'images ultrasonores, tandis que la sonde à ultrasons portative se trouve dans une deuxième position correspondant à une deuxième vue de l'anatomie du patient ; et
commander le réseau de transducteurs afin d'obtenir une troisième pluralité d'images ultrasonores, tandis que la sonde à ultrasons portative se trouve dans une troisième position correspondant à une troisième vue de l'anatomie du patient.

15. Système ultrasonore (100) destiné à guider un utilisateur de manière à obtenir des images ultrasonores optimisées, comprenant :
une sonde à ultrasons portative (110) comprenant un réseau de transducteurs (112) ;
un écran (132) ;
une mémoire (138) ; et
un processeur (116) configuré pour communiquer avec le réseau de transducteurs, l'écran et la mémoire, dans lequel le processeur est configuré pour :
commander le réseau de transducteurs afin d'obtenir une première pluralité d'images ultrasonores correspondant à une ou plusieurs vues de l'anatomie d'un patient ;
calculer une mesure de raccourcissement correspondant à une ou plusieurs images ultrasonores parmi la pluralité d'images ultrasonores ;
afficher à l'écran un indicateur de raccourcissement représentatif de la mesure de raccourcissement ;
comparer la mesure de raccourcissement à un ou plusieurs critères prédéterminés ; et
en réponse au fait que la mesure de raccourcissement ne satisfait pas les un ou plusieurs critères prédéterminés, afficher de manière itérative des instructions destinées à l'utilisateur pour régler l'orientation de la sonde à ultrasons portative, obtenir une pluralité supplémentaire d'images ultrasonores, calculer une mesure de raccourcissement supplémentaire correspondant à la pluralité supplémentaire d'images ultrasonores, mettre à jour l'indicateur de raccourcissement en fonction de la mesure de raccourcissement supplémentaire et comparer la mesure de raccourcissement supplémentaire aux un ou plusieurs critères prédéterminés jusqu'à ce que la mesure de raccourcissement mise à jour satisfasse aux un ou plusieurs critères prédéterminés.
